# EUROPEAN PATENT APPLICATION

(11) **EP 0 856 311 A1**
(43) Date of publication of application: **05.08.1998**
(21) Application number: 96119801.7
(22) Date of filing: 10.12.1996
(51) Int. Cl.: A61K 9/70, A61K 31/21

(54) **Transdermal drug delivery system for the treatment of heart diseases**

(71) Applicant: Rotta Research B.V., 1017 PS Amsterdam (NL)
(72) Inventor: Cordes, Günter, 42799 Leichlingen (DE); Santoro, Antonino, 20052 Monza (IT); Setnikar, Ivo, 20052 Monza (IT)
(74) Representative: Boeters, Hans Dietrich, Dr.

(57) **Abstract**

The invention concerns a transdermal drug delivery system (TDDS) for the delivery of nitroglycerin (NG) to humans, which comprises in accordance with the following sequence at least the following three layers,
(i) a drug free backing layer,
(ii) an optionally cross-linked acrylic adhesive layer having a content of NG and optionally a tackifying agent (tackifyer, tackifying resin or resin), and
(iii) an optionally cross-linked acrylic adhesive layer having a content of NG and a tackifying agent,
and, in addition, a release liner.

## Description

This invention describes a transdermal drug delivery system (TDDS) for the delivery of nitroglycerin to humans for the treatment of heart diseases such as angina pectoris, myocardial infarction, cardiac insufficiency and the like. As nitroglycerin containing TDDS are used for short duration of action the invention describes a system that is delivering a loading dose for immediate action and a controlled release dose for continuous release. The TDDS is a pharmaceutical preparation in the form of a pressure sensitive adhesive matrix patch, consisting of a nitroglycerin impermeable backing foil, nitroglycerin loaded pressure sensitive adhesive layers, and a siliconised protective liner that is discarded upon application of the TDDS to the patient.
Nitroglycerin is widely used in the therapy and prophylaxis of angina pectoris, myocardial infarction paroxysm, cardiac insufficiency and the like. Its effects include dilation of the coronary artery and dilation of collateral and resistance vessels thereby increasing the oxygen supply to the ischaemic area of cardiac muscle.
In the past nitroglycerin has been applied as intravenous infusion, sublingual tablets, soft gelatin capsules, and ointments. For several years transdermal systems have been available for chronic treatment of the above mentioned therapies or prophylaxis.
Anginal heart attacks in general follow a circadian rhythm. The frequency of heart attacks rises in the early morning hours during the wake-up period. At that times an oral nitroglycerin spray is used normally. Then the incidence of attacks is lowered to rise again in the early afternoon hours, to decline again towards the evening and night time. It would be advantageous to have one drug formulation that includes a loading dose to protect the patients immediately after application and to maintain the effective plasma levels of nitroglycerin during the day by means of a controlled release dose.
The duration of action of nitroglycerin is limited by tachyphylaxis. After several hours of continuous input, either as infusion or transdermal application, the intensity of action diminishes. As a consequence, the drug input must be interrupted in order to gain efficacy of the drug again. Nitroglycerin is easily absorbed through the skin. In order to achieve the effective use of the pharmacological effects of nitroglycerin, continuous percutaneous absorption by which a blood level of 0.05 to 2 ng/ml is maintained, is preferable. As the nitroglycerin TDDS are worn for a relatively short time of less than 24 hours it is very important to reach effective plasma levels quickly.
In general, blood levels of drugs after percutaneous absorption show lag times in which effective levels are not reached.

### Previous state of the Art

Numerous patents have been issued for the delivery of nitroglycerin to the systemic circulation via the transdermal route.
In general two types of systems have been described, membrane containing reservoir systems and matrix systems.
According to a representative of the first type of systems nitroglycerin is dissolved in an ethanolic gel, forming the content of the reservoir. The reservoir is covered with an impermeable backing foil and on the other side with a semipermeable membrane onto which a layer of a pressure sensitive adhesive is coated, forming the skin contact layer.
The semipermeable membrane controls the release of nitroglycerin as well as that of ethanol that acts as an absorption enhancer. Disadvantages of this type of systems are described manifold and include skin irritation caused by the ethanol, expensive manufacturing, and instability of the absorption profile.
The newly developed systems are matrix systems, i.e. the nitroglycerin is dispersed within a polymer layer that acts as the adhesive layer, a controlling membrane is omitted. DE 3315272 discribes a multilayer matrix system that maintains a time constant release by varying the drug concentration of the layers, beeing lowest at the skin surface, highest underneath the impermeable backing foil. The TDDS according the present invention does not use this concept, because the present invention intends to exhibit a non-constant drug release by using several layers.
More simple systems are composed of - in general - two layers, the drug impermeable backing layer and the drug containing pressure sensitive adhesive matrix. These systems may contain additional absorption enhancers or in order to improve the cohesion of the matrix, additional cross linkers. If the cohesion of the polymer matrix is too low the matrix may exhibit an undesired creeping tendency that may lead to an adhesion of the cutted edges to the packaging pouch. This may lead to difficulties during the removal of the TDDS from the package often resulting in destruction of the system by the patient.
WO 86/00814, US 5,186,938 describe a partially cross linked matrix system that maintains its integrity during storage or wearing on the skin. It is described in this patent application that the sufficient amount of cross linker preventing the dissolution of the system may lead to a loss of adhesive properties, whereas the adhesivity was good when the systems nearly loses its integrity. It contains 0.2% - 1% cross linker.
The disadvantage of this system is its sensitivity to small concentration changes of cross linker; too much means unsecure adhesion and loss of the patch during the application period, too low concentration means loss of patch integrity leading to a dissolution of the adhesive. This system does not include a loading dose, thus being not able to exhibit a quick onset of action.

European Patent EP 219 539 describes a nitroglycerin patch composed of an acrylic acid polymer pressure sensitive adhesive without cross linker, 10 to 45% nitroglycerin, and a combination of ethyl oleate and glyceryl monolaurate as synergistic absorption enhancers. The patent does not disclose any information on the adhesive properties or the pharmacokinetic behaviour. From the WO 86/00814 can be concluded that nitroglycerin as a solvent like acting drug will soften the adhesive so that the polymer may be dissolved, especially in high concentrations of nitroglycerin. The absorption enhancers also act like plasticisers so that the cohesion of the drug containing polymer matrix will be further decreased. In general, absorption enhancers promote the permeation of drugs through the skin by disturbing the structure of the stratum corneum. Thus, they may cause lokal skin irritations. It will be advantageous to avoid absorption enhancers in order to maintain the integrity of the skin. A patch according to this patent will not be able to exhibit a quick onset of action.
EP 285550 describes a matrix patch, containing nitroglycerin in a concentration of 5 to 20% in an acrylic pressure sensitive adhesive. The polymer is not cross linked. As a consequence the drug load must be relatively low because of the solvent properties of nitroglycerin that may lead to a dissolution of the adhesive. This invention does not consider the pharmacokinetic needs and thus does not include a biphasic absorption profile.
EP 450986 describes an improved patch founded on the invention described in EP 285550, in that an absorption enhancer, a fatty acid alkylester, and for an improvement of cohesivity -colloidal hydrophobic silicid acid anhydride - is added.
EPA 464573 describes a composition of acrylic adhesives containing plasticising drugs and/or absorption enhancers. It is said that cross linking is disadvantageous because the concentration of the cross linker cannot be adjusted sufficiently to the needs of the softening agents. Therefore a non adhesive polymer, increasing the consistency of the matrix is added. This film forming polymer reduces the adhesivity of the matrix. Consequently a tackifyer has to be added that may be a resin derivative. This invention also does not consider the pharmacokinetic needs.
EP 588839 describes an acrylic adhesive based matrix patch containing nitroglycerin. The adhesive has to be cross linked like described in WO 86/00814. No additional advantage over this invention can be deduced from the patent. The patch described in EP 588839 is composed from only one layer containing the nitroglycerin. Thus, no loading dose is effective as can be seen in the in vitro release profiles enclosed in the patent. The inventors have not considered the therapeutic neccessary release profile.
EPA 622075 describes a self cross-linking acrylic adhesive matrix containing drugs like nitroglycerin. The goals of the invention are identical to that of WO 86/00814, US 5,186,938 and EP 588839. The inventors state that the cross linking takes place in the temperature range of 20 - 65°C and that in this range the evaporation of nitroglycerin during the drying process is low. It cannot be seen that there is any advantage over the EP 588839 because acrylic adhesives of the latter patent are cross linking in the same temperature range.

In the light of the aforementioned art there is a need for an optimal transdermal drug delivery system for nitroglycerin.
Therefore a first objective of this present invention is to achieve the quick onset of effective blood levels by use of an effective loading dose.
A second objective of the present invention is to maintain effective plasma levels, after the loading dose has been exhausted, up to 24 hours.
A third objective of the present invention is to make available a transdermal drug delivery system with good skin compatibility.
A fourth objective of the present invention is to make available a TDDS with optimal adhesive properties.
A fifth objective of the present invention is to maintain the integrity of the matrix while having a high drug load in order to avoid absorption enhancers.

A sixth objective of the present invention is to have a TDDS with good physical stability, i.e. small tendency of creeping of the matrix in order to remove the systems easily from the package.

### Description of the invention

The problem underlying the invention is solved by a transdermal drug delivery system (TDDS) for the delivery of nitroglycerin (NG) to humans, which comprises in accordance with the following sequence at least the following three layers,
(i) a drug free backing layer,
(ii) an optionally cross-linked acrylic adhesive layer having a content of NG and optionally a tackifying agent (tackifyer, tackifying resin or resin), and
(iii) an optionally cross-linked acrylic adhesive layer having a content of NG and a tackifying agent,
and, in addition, a release liner.

The TDDS according to the invention can be characterized in that the concentration of NG is 30 to 45 % based on the total weight of layer (ii) and layer (iii).

Further, the TDDS according to the invention can be characterized in that the backing layer consists of polyethylene, polypropylene, polyester, polyurethane or polyvinylchloride.

Further, the TDDS according to the invention can be characterized by a backing layer having a thickness of from 5 to 100 and preferably 10 to 50 µm.

Further, the TDDS according to the invention can be characterized in that the release liner consists of a sheet of a polymer, especially polyethylene, polypropylene, polyester or polyvinylchloride, or paper, or a compound of these materials, especially a polymer-coated paper, preferably a polyethylene coated paper. The polymer can be siliconized.

Further, the TDDS according to the invention can be characterized by a layer of an acrylic adhesive selected from the group of vinylacetate containing co-polymers.

Further, the TDDS according to the invention can be characterized by at least one layer of an acrylic adhesive obtained from glycidyl methacrylate as co-monomer.

Further, the TDDS according to the invention can be characterized by at least one layer of an acrylic adhesive obtained without any use of ethylacrylate as co-monomer.

Further, the TDDS according to the invention can be characterized by a tackifying agent selected from the group consisting of rosin, rosin derivatives, aromatic hydrocarbon resins, aliphatic hydrocarbon resins, terpene resins, and phthalate esters of hydroabietyl alcohol.

Further, the TDDS according to the invention can be characterized by an acrylic adhesive layer (ii) or (iii) cross-linked by a metal chelate, especially titanium acetylacetonide, aluminium acetylacetonide or esters of titanic acid as cross-linker, such as a butyl titanate polymer as titanic acid ester, preferably having a molecular mass of from 750 to 2000 and more preferably of from 1000 to 1500. The concentration of the butyl titanate polymer can be up to 5 % and especially 0.2 to 5 % based on the weight of layer (ii) and/or layer (iii).

Further, the TDDS according to the invention can be characterized in that layer (iii) contains less tackifying agent and more cross-linker than layer (ii).

A specific embodiment of the invention concerns a TDDS characterized in that
= layer (ii) and layer (iii) have about the same NG concentration and that
= layer (iii) has
   - a lower cross-linker concentration and
   - a lower tackifying agent concentration compared with layer (ii); or
   - an approximately corresponding cross-linker concentration and
   - a lower tackifying agent concentration compared with layer (ii).

Further, a specific embodiment of the invention concerns a TDDS characterized in that
= layer (ii) has a lower NG concentration compared with layer (iii) and that
= layer (iii) has
   - an increased cross-linker concentration and
   - a lower tackifying agent concentration compared with layer (ii); or
   - an increased cross-linker concentration and
   - an increased tackifying agent concentration compared with layer (ii).

### Detailed Description of the Invention

Surprisingly it was found that a transdermal system composed of three layers: a drug free backing layer, a first nitroglycerin containing optionally cross-linked acrylic adhesive layer attached to the backing layer, and a second nitroglycerin containing cross-linked acrylic adhesive layer, containing optionally either the same or a different concentration of cross linker as the first layer, and a tackifying agent, with a different or equal nitroglycerin concentration as the first layer, and a release liner, it is possible to get a very quick onset of effective nitroglycerin blood levels, good cohesion of the system so that within 3 years storage at room temperature no adhesion to the packaging material was observed, and excellent skin compatibility.

The backing foil for a nitroglycerin TDDS according to the present invention can be any occlusive material with a thickness of 10 to 50 µm like polyethylene, polypropylene, polyester, and the like, but preferably polyester materials. The release liner is that part of the transdermal drug delivery system that is removed by the patient before application and discarded. The release liner my be any siliconised sheet of material, polyethylene, polypropylene, polyester, polyvinylchloride, paper or a combination of materials like siliconised polyethylene coated paper where the siliconisation is made on the polyethylene layer. Such a compound release liner has the advantage to be rigid for easy removability with a low thickness of polyethylene layer.
A major problem during the development process of the nitroglycerin TDDS was the reliable anchoring of the drug containing matrix to the backing foil.
Acrylic pressure sensitive adesives are known to exhibit excellent adhesive properties. According Donatas Satas (ed.), Handbook of Pressure Sensitive Adhesives, Van Nostrand, 2nd ed., 1989, p.396 ff., no additional tackifying resins are neccessary unless one uses non adhesive acrylates or adhesives for high temperature application.
Upon cross-linking of the matrix the adhesivity decreases so that often the backing foil is peeled off by the patient seperately after the application period. It was now found that the anchoring properties could be optimised by adding a resin into the polymer matrix and by using an acrylic copolymer containing a small amount of glycidyl methacrylate . Thus, the cohesivity of the matrix could be increased in order to minimise the creep. In this layer the main amount of nitroglycerin is present that assures the maintenance of effective plasma levels for a time period up to 24 hours. The tackifying resins may be chosen from the group of rosin and its derivatives, aromatic or aliphatic hydrocarbon resins, terpene resins, but preferable phthalate esters of hydroabietyl alcohol.
The cross linkers used herein may be metal chelates like titanium or aluminium acetylacetonide or esters of titanic acid. Preferably butyltitanate polymer with a molecular mass of approx. 1000 to 1500 is used.
The acrylate pressure sensitive adhesives were chosen from the group of vinylacetate containing copolymers. It was found that adding small amounts of glycidyl methacrylate monomer during the polymerisation of the polymer was very effective for the overall adhesivity in combination with the resin. The polymer should contain no ethyl acrylate. The second nitroglycerin containing layer, being in direct contact to the skin, has a higher drug concentration and may have different concentrations of resin and cross linker, according to the needs of the matrix in order to fulfill its desired function. In general, the resin concentration may be reduced, compared with the first layer, and the cross linker concentration may be increased because of the plasticising action of nitroglycerin. With this system of variable cross linker and tackifyer concentrations it is possible to balance the overall cohesivity of a transdermal system and the ratio of adhesivity to skin and backing foil. The adhesion, expressed as peel force can be adjusted to the type of backing foil, without affecting the peel force on the skin and vice versa.
The ratio of drug concentrations in the first and second matrix layer determines in combination with the thickness of each layer and the degree of cross linking the absorption profile of the nitroglycerin. Cross linking will lead to a "harder" polymer in which the diffusion coefficient of substances will be decreased. If the second - skin contacting - matrix layer has the same nitroglycerin concentration as the first layer, but they differ in the concentration of the cross linker so that the concentration of for example polybutyl titanate is lower in the second layer the drug dissolved in this layer will diffuse faster into the skin than from the first layer. A control of drug release can be achieved by this variation. The effect can be increased further when the second layer has a higher drug concentration than the second. According Fick's diffusion laws the diffusion of a substance through a membrane like skin is determined by the concentration difference on the opposite surfaces of the membrane. Accordingly, a higher concentration of nitroglycerin in the second layer than in the first layer may lead to a higher initial flux until the concentrations in both layers have been equilibrated. Clearly, a blood level profile obtained from this composition is advantageous over the reservoir system when a quick onset of action is needed, for instance in the early morning hours after wake up when the patients have a higher incidence of heart attacks.

The following examples will demonstrate the manufacturing of nitroglycerin TDDS:

### Example 1

### 1. Manufacturing of layer 1

78.09 kg of a solution of an acrylic copolymer composed of 2-hydroxyethylacrylate, vinylacetate as the main monomers, and a small amount of glycidyl methacrylate (pressure sensitive adhesive) in ethylacetate (solid content 33 %) is mixed with 511 g polybutyl titanate (cross-linker), 2.9 kg Cellolyn 21 E (abietyl resin), and 18.75 kg nitroglycerin. The solution is mixed thoroughly and air bubbles are removed. The solution is spread on a siliconised polyester foil of 50 µm thickness, acting as an intermediate liner, to give a surface weight of 48.25 g/m². The wet adhesive layer is dried in a suitable drying oven with increasing temperature and after drying the matrix is covered with a polyester foil (backing foil) of 15 µm thickness. The resulting laminate is wound up to rolls.

### 2. Manufacturing of layer 2

27.75 kg of a solution of an acrylic pressure sensitive adheseve, as mentioned above, is mixed with 171 g polybutyl titanate, 410 g Cellolyn 21 E, and 6.25 kg nitroglycerin. The solution is mixed thoroughly and air bubbles are removed. The solution is spread on a siliconised polyethylene/paper release liner to give a surface weight of 16.08 g/m². The wet adhesive layer is dried in a suitable drying oven with increasing temperature and after drying the matrix is covered with an intermediate liner. The resulting laminate is wound up to rolls.

### 3. Manufacturing of the final laminate

In a suitable lamination machine the roll of layer 2 is unwound and the intermediate liner is removed. Parallely the layer 1 is unwound in the same machine, the intermediate liner is removed, and the two adhesive layers are pressed against each other to give the final laminate composed of a backing foil, two drug containing adhesive layers and a release liner. Th laminate is wound up to a roll.
This example describes a laminate in which the adhesive layer being near the backing foil has an increased resin concentration. The increased resin concentration assures an optimal contact of the drug containing cross-linked matrix to the backing foil. Each of the drug and cross linker concentrations are identical in both adhesive layers.

### 4. Manufacturing of the nitroglycerin TDDS

The laminate is eventually slitted into small rolls according to the size of a TDDS. The small rolls are fed into a suitable die cutting machine where the patches are cut to their final shape and size, 12.5 or 25 cm², releasing 5 or 10 mg nitroglycerin/24 hours, and packed into air tight pouches.

### Example 2

### 1. Manufacturing of layer 1

83.67 kg of a solution of the acrylic copolymer of example 1 is mixed with 610 g polybutyl titanate, 3.0 kg Cellolyn 21 E , and 20.0 kg nitroglycerin. The further steps are analog to those of example 1.

### 2. Manufacturing of layer 2

22.35 kg of a solution of the acrylic copolymer of example 1 is mixed with 72 g polybutyl titanate, 310 g Cellolyn 21 E , and 5.0 kg nitroglycerin. The further steps are analog to those of example 1.

### 3. Manufacturing of the final laminate

In a suitable lamination machine the roll of layer 2 is unwound and the intermediate liner is removed. Parallely the layer 1 is unwound in the same machine, the intermediate liner removed, and the two adhesive layers are pressed against each other to give the final laminate composed of a backing foil, two drug containing adhesive layers and a release liner. Th laminate is wound up to a roll.
The adhesive layer being near the backing foil has increased resin and cross linker concentrations. As a consequence of a higher degree of cross linking the nitroglycerin containing matrix the adhesion to the backing foil is decreased. Increasing the resin concentration in the first layer adjusts the adhesion to form the optimal bond to the foil. The reduced cross linker in the second layer may lead to a higher diffusivity of nitroglycerin in this matrix.

### 4. Manufacturing of the nitroglycerin TDDS

The laminate is eventually slitted into small rolls according to the size of a TDDS. The small rolls are fed into a suitable die cutting machine where the patches are cut to their final shape and packeged into air tight pouches.

### Example 3

### 1. Manufacturing of layer 1

97.26 kg of a solution of the acrylic copolymer of example 1 is mixed with 590 g polybutyl titanate, 3.11 kg Cellolyn 21 E , and 21.78 kg nitroglycerin. The further steps are analog to those of example 1.

### 2. Manufacturing of layer 2

8.76 kg of a solution of the acrylic copolymer of example 1 is mixed with 92 g polybutyl titanate, 200 g Cellolyn 21 E , and 3.22 kg nitroglycerin. The further steps are analog to those of example 1.

### 3. Manufacturing of the final laminate

In a suitable lamination machine the roll of layer 2 is unwound and the intermediate liner is removed. Parallely the layer 1 is unwound in the same machine, the intermediate liner is removed, and the two adhesive layers are pressed against each other to give the final laminate composed of a backing foil, two drug containing adhesive layers and a release liner. The laminate is wound up to a roll.
The adhesive layer being near the backing foil has increased resin and reduced cross linker and nitroglycerin concentrations, whereas the layer 2, being in contact with the release liner has increased nitroglycerin and cross linker concentrations. The second layer, being in contact with the skin after application of the transdermal system, contains a higher nitroglycerin concentration than the first layer, resulting in a faster release of a part of the whole nitroglycerin dose. Due to the softening effect of the nitroglycerin the cross linker concentration has to be increased additionally.

### 4. Manufacturing of the nitroglycerin TDDS

The laminate is eventually slitted into small rolls according to the size of a TDDS. The small rolls are fed into a suitable die cutting machine where the patches are cut to their final shape and packeged into air tight pouches.

### Example 4

### 1. Manufacturing of layer 1

13.46 kg of a solution of the acrylic copolymer of example 1 is mixed with 52 g polybutyl titanate, 150 g Cellolyn 21 E , and 4.71 kg nitroglycerin. The further steps are analog to those of example 1.

### 2. Manufacturing of layer 2

87.51 kg of a solution of the acrylic copolymer of example 1 is mixed with 630 g polybutyl titanate, 3.16 g Cellolyn 21 E , and 23.5 kg nitroglycerin. The further steps are analog to those of example 1.

### 3. Manufacturing of the final laminate

In a suitable lamination machine the roll of layer 2 is unwound and the intermediate liner is removed. Parallely the layer 1 is unwound in the same machine, the intermediate liner is removed, and the two adhesive layers are pressed against each other to give the final laminate composed of a backing foil, two drug containing adhesive layers and a release liner. The laminate is wound up to a roll.
The adhesive layer being near the backing foil has reduced resin, reduced cross linker and reduced nitroglycerin concentrations, whereas the layer 2, being in contact with the release liner has increased nitroglycerin, cross linker, and resin concentrations. The second layer, being in contact with the skin after application of the transdermal system, assures a quick and long onset and the first layer contains a small maintenence dose. Because of the reduced nitroglycerin concentration the cross linker and the resin concentrations may be lower because of the lack of softening effect of the drug.

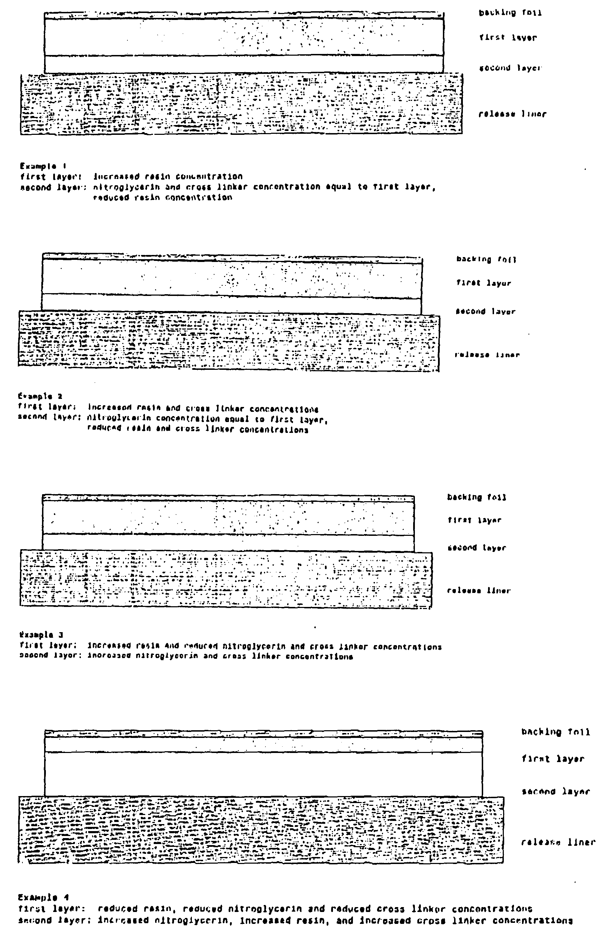

## Claims

1. Transdermal drug delivery system (TDDS) for the delivery of nitroglycerin (NG) to humans, comprising in accordance with the following sequence at least the following three layers,
(i) a drug free backing layer,
(ii) an optionally cross-linked acrylic adhesive layer having a content of NG and optionally a tackifying agent (tackifyer, tackifying resin or resin), and
(iii) an optionally cross-linked acrylic adhesive layer having a content of NG and a tackifying agent,
and, in addition, a release liner.

2. TDDS according to claim 1, **characterized** in that the concentration of NG is 30 to 45 % based on the total weight of layer (ii) and layer (iii).

3. TDDS according to claim 1 or 2, **characterized** in that the backing layer consists of polyethylene, polypropylene, polyester, polyurethane or polyvinylchloride.

4. TDDS according to any of the preceding claims, **characterized** by a backing layer having a thickness of from 5 to 100 and preferably 10 to 50 µm.

5. TDDS according to any of the preceding claims, **characterized** in that the release liner consists of a sheet of a polymer, especially polyethylene, polypropylene, polyester or polyvinylchloride, or paper, or a compound of these materials, especially a polymer-coated paper, preferably a polyethylene coated paper.

6. TDDS according to claim 5, **characterized** in that the polymer is siliconized.

7. TDDS according to any of the preceding claims, **characterized** by a layer of an acrylic adhesive selected from the group of vinylacetate containing co-polymers.

8. TDDS according to any of the preceding claims, **characterized** by at least one layer of an acrylic adhesive obtained from glycidyl methacrylate as co-monomer.

9. TDDS according to any of the preceding claims, **characterized** by at least one layer of an acrylic adhesive obtained without any use of ethylacrylate as co-monomer.

10. TDDS according of any of the preceding claims, **characterized** by a tackifying agent selected from the group consisting of rosin, rosin derivatives, aromatic hydrocarbon resins, aliphatic hydrocarbon resins, terpene resins, and phthalate esters of hydroabietyl alcohol.

11. TDDS according to any of the preceding claims, **characterized** by an acrylic adhesive layer (ii) or (iii) cross-linked by a metal chelate, especially titanium acetylacetonide, aluminium acetylacetonide or esters of titanic acid as cross-linker.

12. TDDS according to claim 11, **characterized** by a butyl titanate polymer as titanic acid ester, preferably having a molecular mass of from 750 to 2000 and more preferably of from 1000 to 1500.

13. TDDS according to claim 11 or 12, **characterized** by a butyl titanate polymer as titanic acid ester having a concentration of up to 5 % and especially 0.2 to 5 % based on the total weight of layer (ii) and/or layer (iii).

14. TDDS according to any of the preceding claims, **characterized** in that layer (iii) contains less tackifying agent and more cross-linker than layer (ii).

15. TDDS according to any of the preceding claims, **characterized** in that
= layer (ii) and layer (iii) have about the same NG concentration and that
= layer (iii) has
- a lower cross-linker concentration and
- a lower tackifying agent concentration compared with layer (ii); or
- an approximately corresponding cross-linker concentration and
- a lower tackifying agent concentration compared with layer (ii).

16. TDDS according to any of the claims 1 to 14, **characterized** in that
= layer (ii) has a lower NG concentration compared with layer (iii) and that
= layer (iii) has
- an increased cross-linker concentration and
- a lower tackifying agent concentration compared with layer (ii); or
- an increased cross-linker concentration and
- an increased tackifying agent concentration compared with layer (ii).
